(19) Europäisches Patentamt
European Patent Office
Office européen des brevets

(11) EP 0 870 495 A2

(12) # EUROPÄISCHE PATENTANMELDUNG

(43) Veröffentlichungstag:
**14.10.1998 Patentblatt 1998/42**

(51) Int. Cl.[6]: **A61K 7/50**, A61K 7/00

(21) Anmeldenummer: **98105312.7**

(22) Anmeldetag: **24.03.1998**

(84) Benannte Vertragsstaaten:
**AT BE CH DE DK ES FI FR GB GR IE IT LI LU MC NL PT SE**
Benannte Erstreckungsstaaten:
**AL LT LV MK RO SI**

(30) Priorität: **10.04.1997 DE 19714829**

(71) Anmelder:
**Beiersdorf Aktiengesellschaft**
**20245 Hamburg (DE)**

(72) Erfinder:
- **Lerg, Heike**
  **22303 Hamburg (DE)**
- **Schmucker, Robert, Dr.**
  **22523 Hamburg (DE)**
- **Müller, Anja**
  **23843 Rümpel (DE)**
- **Gers-Barlag, Heinrich, Dr.**
  **25495 Kummerfeld (DE)**

(54) **Kosmetische Reinigungsmittel auf der Grundlage von Emulsionen, die keinen Emulgator im herkömmlichen Sinn enthalten**

(57) Verwendung von Mischungen, die bezogen auf das Gesamtgewicht der Zubereitungen

1 bis 50 Gew.-% eines oder mehrerer Tenside und
5 bis 60 Gew.-% Wasser und
25 bis 90 Gew.-% einer oder mehrerer Ölkomponenten sowie

gegebenenfalls weitere kosmetische oder pharmazeutische Hilfs-, Zusatz- und/oder Wirkstoffe enthalten, als kosmetische und/oder dermatologische Reinigungszubereitungen.

EP 0 870 495 A2

## Beschreibung

Die vorliegende Erfindung betrifft kosmetische Reinigungsmittel auf der Grundlage von Emulsionen, die keinen Emulgator im herkömmlichen Sinn enthalten.

Reinigung ist die Basis jeder kosmetischen Anwendung. Sie bedeutet das Entfernen von (Umwelt-) Schmutz, Hautschuppen, Schweißrückständen und Keimen und bewirkt damit subjektiv eine Erhöhung des psychischen und physischen Wohlbefindens.

Kosmetische Reinigungsmittel werden dem Verbraucher in Form von verschiedenen Zubereitungen angeboten. Zubereitungen dieser Art sind beispielsweise Schaum- und Duschbäder, feste und flüssige Seifen oder sogenannte "Syndets" (synthetische Detergentien). Shampoos, Handwaschpasten, Intimwaschmittel, spezielle Reinigungsmittel für Kleinkinder und dergleichen.

In kosmetischen Reinigungsmitteln spielen waschaktive Substanzen, die sogenannten Tenside, eine zentrale Rolle. Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutsentfernung und -lösung, ein leichtes Abspülen und -je nach Wunsch - für Schaumregulierung.

Bei den hydrophilen Anteilen eines Tensidmoleküls handelt es sich meist um polare funktionelle Gruppen, beispielweise $-COO^-$, $-OSO_3^{2-}$, $-SO_3^-$, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:

- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wäßriger Lösung bilden sie im sauren oder neutralen Milieu negativ geladene organische Ionen. Kationische Tenside sind beinahe ausschließlich durch das Vorhandensein einer quarternären Ammoniumgruppe gekennzeichnet. In wäßriger Lösung bilden sie im sauren oder neutralen Milieu positiv geladene organische Ionen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | | |
|---|---|---|
| $RNH_2^+CH_2CH_2COOH\ X^-$ | (bei pH=2) | $X^-$ = beliebiges Anion, z.B. $Cl^-$ |
| $RNH_2^+CH_2CH_2COO^-$ | (bei pH=7) | |
| $RNHCH_2CH_2COO^-\ B^+$ | (bei pH=12) | $B^+$ = beliebiges Kation, z.B. $Na^+$ |

Typisch für nicht-ionische Tenside sind Polyether-Ketten. Nicht-ionische Tenside bilden in wäßrigem Medium keine Ionen.

Bereits bei einer Reinigung der Haut mit Hilfe eines einfachen Wasserbads - ohne Zusatz von Tensiden - kommt es zunächst zu einer Quellung der Hornschicht der Haut. Der Grad dieser Quellung hängt u. a. von der Dauer des Bads und dessen Temperatur ab. Gleichzeitig werden wasserlösliche Stoffe ab- bzw. ausgewaschen, wie z.B. wasserlösliche Schmutzbestandteile, aber auch hauteigene Stoffe, die für das Wasserbindungsvermögen der Hornschicht verantwortlich sind. Durch hauteigene oberflächenaktive Stoffe werden außerdem auch Hautfette in gewissem Ausmaß gelöst und ausgewaschen. Dies bedingt nach anfänglicher Quellung eine nachfolgende Austrocknung der Haut, die durch waschaktive Zusätze noch deutlich verstärkt werden kann.

Bei gesunder Haut sind diese Vorgänge im allgemeinen belanglos, da die Schutzmechanismen der Haut solche leichten Störungen der oberen Hautschichten ohne weiteres kompensieren können. Aber bereits im Fall nichtpathologischer Abweichungen vom Normalstatus, z.B. durch umweltbedingte Abnutzungsschäden bzw. Irritationen, Lichtschäden, Altershaut usw., ist der Schutzmechanismus der Hautoberfläche gestört. Unter Umständen ist er dann aus eigener Kraft nicht mehr imstande, seine Aufgabe zu erfüllen, und muß durch externe Maßnahmen regeneriert werden. Es hat daher nicht an Versuchen gefehlt. geeignete Reinigungszubereitungen zur gleichzeitigen Regeneration der Haut zu finden.

Bekannte Mittel zur Reinigung und gleichzeitigen Pflege der Haut sind flüssige Reinigungsprodukte auf Ölbasis, beispielsweise Ölbäder. Diese zeigen hohe Pflegeeigenschaften, allerdings ist der Einsatz von Tensiden aufgrund ihrer mangelnden Löslichkeit in Öl auf wenige Beispiele beschränkt. Zudem sind Ölbäder praktisch nicht oder allenfalls schwach schäumend, da Lipide die Schaumentwicklung sehr stark herabsetzen.

Auch Reinigungszubereitungen für die Verwendung als Duschpräparat sind an sich bekannt. Es handelt sich dabei im wesentlichen um oberflächenaktive Substanzen oder Stoffgemische, die dem Verbraucher in verschiedenen Zubereitungen angeboten werden. Zubereitungen solcher Art zeichnen sich im allgemeinen durch einen mehr oder weniger hohen Wassergehalt aus, können aber auch beispielsweise als Konzentrat vorliegen.

Im allgemeinen unterscheiden sich Präparate, welche für das Duschbad vorgesehen sind, nicht oder kaum von Wannenbadzubereitungen, abgesehen davon, daß bei Duschzubereitungen Produkte höherer Viskosität bevorzugt werden, die nach Entnahme aus dem Behälter nicht aus der Hand rinnen. Dies ist bei Wannenbadzubereitungen weniger von praktischer Bedeutung.

Ein erheblicher Nachteil von Wannenbadzubereitungen ist, daß sie in sehr großer Verdünnung vorliegen, da der Inhalt einer Badewanne im Einzelfall bis zu mehreren hundert Litern Wasser betragen kann. Dem muß durch besondere Formulierungssorgfalt bzw. Anwendung großer Mengen der zu verwendenden Ölbadzubereitung Rechnung getragen werden.

Tensidhaltige Duschzubereitungen entfalten im allgemeinen keine nennenswerte Pflegewirkung, da sie nur einen geringen Ölgehalt aufweisen. Die nicht anwendungsgerechte Nutzung einer Ölbadzubereitung als Duschpräparat ist da keine Alternative. Diese Nutzung ist unzweckmäßig, da solche Zubereitungen keinen nennenswerten Schaum entwickeln.

Eine relativ neue technische Entwicklung sind hingegen tensidhaltige Duschzubereitungen mit hohem Ölgehalt. Die Deutsche Offenlegungsschrift 44 24 210 beschreibt in diesem Zusammenhang kosmetische oder dermatologische Duschzubereitungen mit einem Tensidgehalt von höchstens 55 Gew.-% und einem Ölgehalt von mehr als 45 Gew.-%, wobei die Zubereitungen im wesentlichen wasserfrei sind. Aufgrund des hohen Ölgehalts wirken diese Zubereitungen regenerierend in bezug auf den allgemeinen Hautzustand. Sie haben dabei gleichzeitig eine gute Schaumentwicklung und eine hohe Reinigungskraft.

Der Stand der Technik kennt zur Reinigung und gleichzeitigen Pflege der Haut ferner Reinigungsprodukte auf Emulsionsbasis. Diese werden in der Art formuliert, daß die Emulsion mit Emulgatoren stabilisiert und anschließend ein Tensidsystem angepaßt wird. Da Emulsionen durch die Zugabe von Tensiden im allgemeinen zerstört werden, ist die Wahl des Tensidsystems stark eingeschränkt, und den erhaltenen Reinigungszubereitungen liegen teure und komplizierte Rezepturen zugrunde.

Kosmetische Reinigungsmittel enthalten meist Mischungen von Tensiden verschiedener Art. Die Auswahl orientiert sich in erster Linie an der Hautverträglichkeit und der gewünschten kosmetischen Leistung der Tenside. Daneben spielen Schaumvermögen, Formulierbarkeit und ein günstiges Leistungs-/Kostenverhältnis eine wesentliche Rolle.

Das wohl bekannteste Beispiel einer waschaktiven Substanz ist die klassische Seife. Seife entsteht bei der chemischen Reaktion eines Fettes oder daraus gewonnener Fettsäuren bzw. Fettsäureestern mit Natron- oder Kalilauge, wobei die Qualität der Ausgangsfette für die Güte der daraus gewonnenen Seife mitbestimmend ist. Wichtig für die Auswahl der Fette ist ferner die Verteilung der Kettenlängen der entsprechenden Fettsäuren.

Das Optimum an Schaumbildungs- und Reinigungsvermögen zeigen Tenside, deren lipophiler Molekülbereich eine Kettenlänge von zwölf Kohlenstoffatomen aufweist. die also durch Verseifung von Laurinsäure (-derivaten) gewonnen wurden. Tenside auf der Grundlage von Laurinsäure (-derivaten) werden daher bevorzugt in Reinigungszubereitungen, sogenannten „rinse-off"-Produkten verwendet, die nach der Anwendung von der Haut abgespült werden.

Auch Emulgatoren haben eine amphiphile Struktur, sind also den Tensiden von der Struktur her vergleichbar. Emulgatoren ermöglichen oder erleichtern die gleichmäßige Verteilung zweier oder mehrerer nicht mischbarer Phasen und verhindern gleichzeitig deren Entmischung. Sie werden daher zur Stabilisierung von Wasser-Lipid-Mischungen in Form von Emulsionen eingesetzt. Emulgatoren setzen die Grenzflächenspannung zwischen den Phasen herab, indem sie an der Phasengrenze Öl/Wasser Grenzflächenfilme ausbilden, wodurch dem irreversiblen Zusammenfließen der Tröpfchen entgegengewirkt wird. Zur Stabilisierung von Emulsionen werden häufig Emulgatorgemische verwendet.

Eine optimale Wechselwirkung zwischen Emulgator und Öl, d. h. eine hohe Stabilität der Emulsion, wird erreicht, wenn die Kettenlänge des lipophilen Emulgatorbereichs der Fettsäure-Kettenlänge der eingesetzten Öle entspricht. Emulgatoren im herkömmlichen Sinn haben daher - im Unterschied zu den im allgemeinen verwendeten Tensiden - eine Kettenlänge von mehr als 16, im allgemeinen zwischen 16 und 20 Kohlenstoffatomen.

Emulgatoren werden bevorzugt in kosmetischen oder dermatologischen Formulierungen, sogenannten „leave-on"-Produkten, eingesetzt, die nach der Anwendung auf der Haut verbleiben, welche also beispielsweise zur Behandlung und Pflege der Haut dienen.

Aufgabe der vorliegenden Erfindung war es, Reinigungszubereitungen auf der Grundlage von Emulsionen zur Verfügung zu stellen, welche den Nachteilen des Standes der Technik Abhilfe schaffen und denen dementsprechend einfache und kostengünstige Rezepturen zugrunde liegen. Die Zubereitungen sollten zudem eine hohe Pflegewirkung besitzen, ohne daß die reinigende Wirkung dahinter zurücksteht.

Die vorliegende Erfindung betrifft ferner waschaktive haarkosmetische Zubereitungen, im allgemeinen als Shampoos bezeichnet. Insbesondere betrifft die vorliegende Erfindung haarkosmetische Wirkstoffkombinationen und Zubereitungen zur Pflege des Haars und der Kopfhaut. Dem Stand der Technik mangelt es an Shampooformulierungen, welche geschädigtem Haar in befriedigender Weise Pflege zukommen lassen. Aufgabe war daher, auch diesen Nachteilen des Stands der Technik Abhilfe zu schaffen.

Es hat sich überraschend gezeigt, und darin liegt die Lösung dieser Aufgaben, daß die Verwendung von Mischungen, die bezogen auf das Gesamtgewicht der Zubereitungen

1 bis 50 Gew.-% eines oder mehrerer Tenside und
5 bis 60 Gew.-% Wasser und
25 bis 90 Gew.-% einer oder mehrerer Ölkomponenten sowie

gegebenenfalls weitere kosmetische oder pharmazeutische Hilfs-, Zusatz- und/oder Wirkstoffe enthalten, als kosmetische und/oder dermatologische Reinigungszubereitungen den Nachteilen des Standes der Technik abhilft.

Den kosmetischen und/oder dermatologischen Reinigungsemulsionen im Sinn der vorliegenden Erfindung liegen einfache und kostengünstige Rezepturen zugrunde. Sie haben gleichzeitig eine gute Schaumentwicklung und eine hohe Reinigungskraft. Aufgrund des hohen Ölgehalts wirken diese Zubereitungen regenerierend in bezug auf den allgemeinen Hautzustand, vermindern das Trockenheitsgefühl der Haut und machen die Haut geschmeidig.

Die Reinigungsemulsionen enthalten im Sinn der vorliegenden Erfindung vorteilhaft eines oder mehrere Tenside der folgenden vier Gruppen A bis D:

A. Anionische Tenside

Vorteilhaft zu verwendende anionische Tenside sind

Acylaminosäuren (und deren Salze), wie

1. Acylglutamate, beispielsweise Natriumacylglutamat, Di-TEA-palmitoylaspartat und Natrium Caprylic/ Capric Glutamat,
2. Acylpeptide, beispielsweise Palmitoyl hydrolysiertes Milchprotein, Natrium Cocoyl hydrolysiertes Soja Protein und Natrium-/ Kalium Cocoyl hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,

Carbonsäuren und Derivate, wie

1. Carbonsäuren beispielsweise Laurinsäure, Aluminiumstearat, Magnesiumalkanolat und Zinkundecylenat,
2. Ester-Carbonsäuren, beispielsweise Calciumstearoyllactylat, Laureth-6 Citrat und Natrium PEG-4 Lauramidcarboxylat,
3. Ether-Carbonsäuren, beispielsweise Natriumlaureth-13 Carboxylat und Natrium PEG-6 Cocamide Carboxylat,

Phosphorsäureester und Salze, wie beispielsweise DEA-Oleth-10 Phosphat und Dilaureth-4 Phosphat,

Sulfonsäuren und Salze, wie

1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium $C_{12-14}$ Olefinsulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat

sowie

Schwefelsäureester, wie

1. Alkylethersulfat, beispielsweise Natrium-, Ammonium-, Magnesium-, MIPA-, TIPA- Laurethsulfat, Natriummyrethsulfat und Natrium $C_{12-13}$ Parethsulfat,
2. Alkylsulfate, beispielsweise Natrium-, Ammonium- und TEA- Laurylsulfat.

B. Kationische Tenside

Vorteilhaft zu verwendende kationische Tenside sind

1. Alkylamine,
2. Alkylimidazole,
3. Ethoxylierte Amine und
4. Quarternäre Tenside.

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind Benzalkoniumchlorid, Alkylbetain, Alkylamidopropylbetain und Alkyl-amidopropylhydroxysultain.

C. Amphotere Tenside

Vorteilhaft zu verwendende amphotere Tenside sind

1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren beispielsweise Aminopropylalkylglutamid, Alkylaminopropionsäure, Natriumalkylimidodipropionat und Lauroamphocarboxyglycinat.

D. Nicht-ionische Tenside

Vorteilhaft zu verwendende nicht-ionische Tenside sind

1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte Alkohole, ethoxyliertes Lanolin, ethoxylierte Polysiloxane, propoxylierte POE Ether und Alkylpolyglycoside wie Laurylglucosid, Decylglycosid und Cocoglycosid.

Die Reinigungsemulsionen enthalten im Sinn der vorliegenden Erfindung besonders vorteilhaft eines oder mehrere Tenside aus den Gruppen der anionischen und/oder amphoteren Tenside.

Vorteilhaft ist ferner die Verwendung einer Kombination von anionischen und/oder amphoteren Tensiden mit einem oder mehreren nicht-ionischen Tensiden.

Die Ölphase der kosmetischen Reinigungsemulsionen wird im Sinn der vorliegenden Erfindung vorteilhaft aus der Gruppe der Lipide gewählt. In der biochemischen Fachsprache werden unter dem Begriff „Lipide“ bestimmte, strukturell an sich durchaus nicht einheitliche Biomoleküle zusammengefaßt. Im ursprünglichen Sinn sind unter „Lipiden" Fette zu verstehen, also Carbonsäureester des Glycerins.

Im weiteren Sinne wird in diesem Begriff eine Gruppe von in Wasser unlöslichen Molekülen verstanden, welche sich durch wenigstens einen ausgeprägt hydrophilen Molekülbereich und wenigstens einen ausgeprägt lipophilen Molekülbereich auszeichnen. Die Phosphorsäureester acylierter Glycerine, die sogenannten „Phospholipide“, und andere Verbindungen gehören zu dieser insgesamt recht inhomogenen Gruppe chemischer Verbindungen.

Von größter Bedeutung unter den Phosphatidylcholinen sind beispielsweise die Lecithine, welche sich durch die allgemeine Struktur

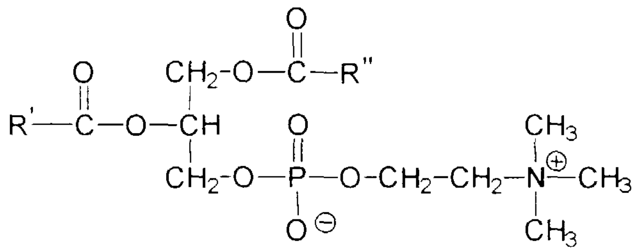

auszeichnen, wobei R' und R" typischerweise unverzweigte aliphatische Reste mit 15 oder 17 Kohlenstoffatomen und bis zu 4 cis-Doppelbindungen darstellen.

Die Ölphase der Reinigungsemulsionen wird im Sinn der vorliegenden Erfindung vorteilhaft gewählt aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z.B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl und dergleichen mehr.

Vorteilhaft werden die Öle ebenfalls gewählt aus der Gruppe Vaseline (Petrolatum), Paraffinöl und Polyolefine. Unter den Polyolefinen sind Polydecene die bevorzugten Substanzen.

Die Ölphase kann im Sinne der vorliegenden Erfindung ferner vorteilhaft gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, wie z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan),

Besonders vorteilhafte Ausführungsformen der erfindungsgemäßen kosmetischen und dermatologischen waschaktiven Zubereitungen, die ihrerseits auf erfinderischer Tätigkert beruhen, enthalten zusätzlich zu der wäßrigen Tensidlösung und der Ölphase einen Gehalt von 0,1 bis 10 Gew.-% an mikronisierten, anorganischen Pigmenten, die gewählt werden aus der Gruppe der Metalloxide und gegebenenfalls oberflächlich behandelt sind.

Vorteilhaft ist es, den mittleren Partikeldurchmesser der verwendeten Pigmente kleiner als 200 nm, besonders vorteilhaft zwischen 1 nm und 200 nm, insbesondere zwischen 5 nm und 50 nm zu wählen. Im wesentlichen unerheblich für die vorliegende Erfindung ist es, in welcher der gegebenenfalls natürlich vorkommenden Modifikationen die verwendeten Metalloxide vorliegen.

Diese Ausführungsformen stellen Abmischungen aus Ölen bzw. öllöslichen Substanzen und Wasser bzw. wasserlöslichen Komponenten dar, die durch den Zusatz von mikronisierten Feststoffpartikeln stabilisiert werden und ebenfalls keinen Emulgator im herkömmlichen Sinn enthalten. Sie werden dadurch stabilisiert, daß sich die Pigmentpartikel an die Tröpfchen der dispersen Phase anlagern und sozusagen eine mechanische Barriere bilden, die das Koaleszieren, d. h. das Zusammenfließen der Tröpfchen verhindert. Derartige Zubereitungen sind an sich bekannt und werden

im allgemeinen als Pickering-Emulsionen bezeichnet.

Die Europäische Offenlegungsschrift 0 686 391 beschreibt beispielsweise Emulsionen vom Typ Wasser-in-Öl, die frei von oberflächenaktiven Substanzen sind und nur durch Feststoffe stabilisiert werden. Zur Stabilisierung werden hier sphärische Polyalkylsilsesquioxan-Partikel eingesetzt, die einen Durchmesser von 100 nm bis zu 20 µm haben.

Pickering-Emulsionen, die feindisperse Systeme vom Typ Öl-in-Wasser oder Wasser-in-Öl darstellen, sind relativ variabel in ihrem Wasser-Fettphasen-Verhältnis und bieten den Vorteil, daß hohe Mengen an Ölen in Wasser stabil eingearbeitet werden können.

Vorteilhaft im Sinne der vorliegenden Erfindung ist es, zur Stabilisierung von Öl-in-Wasser-(O/W-) Pickering-Emulsionen unbehandelte, nahezu reine Pigmentpartikel zu verwenden, insbesondere solche, welche auch als Farbstoff in der Lebensmittelindustrie und/oder als Absorber von UV-Strahlung in Sonnenschutzmitteln verwendet werden können. Vorteilhaft sind beispielsweise Titandioxid-Pigmente und/oder Zinkoxid-Pigmente, die unter den Handelsbezeichnungen KRONOS® 1171 ($TiO_2$) von der Firma Kronos Titan und NanoX (ZnO) von der Harcros Chemical Group erhältlich sind.

Pickering-Emulsionen werden im Sinne der vorliegenden Erfindung besonders vorteilhaft durch anorganische Pigmente stabilisiert die in hydrophober Form vorliegen, d. h., daß sie oberflächlich wasserabweisend behandelt („gecoatet") sind. Diese Oberflächenbehandlung kann darin bestehen, daß die Pigmente nach an sich bekannten Verfahren mit einer dünnen hydrophoben Schicht versehen werden.

Ein solches Verfahren, das im folgenden am Beispiel von Titandioxid beschrieben wird, besteht beispielsweise darin, daß die hydrophobe Oberflächenschicht nach einer Reaktion gemäß

$$n\ TiO_2 + m\ (RO)_3Si\text{-}R' \rightarrow n\ TiO_2\ (\text{oberfl.})$$

erzeugt wird. n und m sind dabei nach Belieben einzusetzende stöchiometrische Parameter, R und R' die gewünschten organischen Reste. Von Vorteil sind u. a. hydrophobisierte Pigmente, die in Analogie zur Deutschen Offenlegungsschrift 33 14 742 dargestellt werden. Besonders vorteilhaft sind $TiO_2$-Pigmente, beispielsweise die unter der Handelsbezeichnung T805 bei der Firma Degussa erhältlichen.

Eine weitere vorteilhafte Beschichtung der anorganische Pigmente besteht aus Dimethylpolysiloxan, einem Gemisch vollmethylierter, linearer Siloxanpolymere, die endständig mit Trimethylsiloxy-Einheiten blockiert sind. Besonders vorteilhaft sind Zinkoxid-Pigmente, die auf diese Weise beschichtet werden.

Vorteilhaft im Sinne der vorliegenden Erfindung ist ferner die Verwendung einer Kombination von Titandioxidpigmenten, die mit Octylsilanol beschichtet sind, und Siliciumdioxidpartikeln, die oberflächlich wasserabweisend behandelt („gecoatet") sind. Solche Siliciumdioxidpartikel sind beispielsweise die in der Europäischen Offenlegungsschrift 0 686 391 erwähnten sphärischen Polyalkylsilsesquioxan-Partikel. Vorteilhafte Ausführungsformen entsprechender Titandioxidpartikel sind unter der Handelsbezeichnung T805 bei der Firma Degussa erhältlich. Vorteilhafte Ausführungsformen entsprechender Polyalkylsilsesquioxan-Partikel sind unter der Handelsbezeichnung Aerosil R972 bei der Firma Degussa erhältlich.

Pickering-Emulsionen vom Typ Öl-in-Wasser (O/W-Pickering-Emulsionen) werden im Sinne der vorliegenden Erfindung besonders vorteilhaft durch Metalloxidpartikel stabilisiert, die mit Aluminiumhydroxid und/oder Siliziumdioxid überzogen („gecoatet") sind. Vorteilhafte Ausführungsformen sind beispielsweise Titandioxidpartikel, die unter dem Namen EUSOLEX® TA bei der Firma Merck erhältlich sind.

Vorteilhaft im Sinne der vorliegenden Erfindung ist ferner die Verwendung einer Mischung verschiedener Pigmenttypen sowohl innerhalb eines Kristalls, beispielsweise als Eisenmischoxid oder Talkum (Magnesiumsilicat), als auch durch Kombination mehrerer Metalloxidtypen innerhalb einer Zubereitung.

Vorteilhaft in allen vorgenannten Fällen ist es, die Konzentration der Pigmente größer als 0.1 Gew.-%, besonders vorteilhaft zwischen 0.1 Gew.-% und 5 Gew.-% bezogen auf das Gesamtgewicht der Zubereitungen zu wählen.

Kosmetische Zubereitungen, die kosmetische Reinigungszubereitungen für die Haut darstellen, können in flüssiger oder fester Form vorliegen. Sie enthalten vorzugsweise mindestens eine anionische, nicht-ionische oder amphotere oberflächenaktive Substanz oder Gemische daraus und Hilfsmittel, wie sie üblicherweise dafür verwendet werden.

Kosmetische Zubereitungen, die ein Shampoonierungsmittel darstellen, enthalten vorzugsweise mindestens eine anionische, nicht-ionische oder amphotere oberflächenaktive Substanz oder Gemische daraus und Hilfsmittel, wie sie üblicherweise dafür verwendet werden. Die oberflächenaktive Substanz kann in einer Konzentration zwischen 1 Gew.-% und 50 Gew.-% in dem Shampoonierungsmittel vorliegen.

Die Zusammensetzungen enthalten gemäß der Erfindung außer den vorgenannten Tensiden gegebenenfalls die in der Kosmetik üblichen Zusatzstoffe, beispielsweise Parfüm, Farbstoffe, antimikrobielle Stoffe, rückfettende Agentien, Komplexierungs- und Sequestrierungsagentien, Perlglanzagentien, Pflanzenextrakte, Vitamine, Wirkstoffe, Konservierungsmittel, Bakterizide, Pigmente, die eine färbende Wirkung haben, Verdickungsmittel, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische

Lösemittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, ψ-Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta- Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes. Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die folgenden Beispiele, in welchen Waschpräparate zur Haar- und Körperpflege beschrieben werden, sollen die erfindungsgemäßen Zusammensetzungen erläutern, ohne daß aber beabsichtigt ist, die Erfindung auf diese Beispiele zu beschränken. Die Zahlenwerte in den Beispielen bedeuten Gewichtsprozente, bezogen auf das Gesamtgewicht der jeweiligen Zubereitungen.

| **Beispiel 1:** | |
|---|---|
| Paraffinöl | 70 |
| Natriumlaurethsulfat (25 %-ige Lösung in Wasser) | 29,5 |
| Parfüm Antioxidantien, Konservierungsstoffe | q.s. |

| **Beispiel 2:** | |
|---|---|
| Paraffinöl | 70 |
| Natrium PEG-4 Lauramidcarboxylat (30 %-ige Lösung in Wasser) | 29,5 |
| Parfüm, Antioxidantien, Konservierungsstoffe | q.s. |

| **Beispiel 3:** | |
|---|---|
| Paraffinöl | 46 |
| Ricinusöl | 24,3 |
| Natriumlaurethsulfat (25 %-ige Lösung in Wasser) | 29,4 |
| Parfüm, Antioxidantien, Konservierungsstoffe | q.s. |

| **Beispiel 4:** | |
|---|---|
| Paraffinöl | 70 |
| Natrium Cocoamphoacetat (30 %-ige Lösung in Wasser) | 29,2 |
| Parfüm, Antioxidantien, Konservierungsstoffe | q.s. |

| **Beispiel 5:** | |
|---|---|
| Natriumlaurethsulfat (25 %-ige Lösung in Wasser) | 49,4 |
| Sojaöl | 36 |
| Paraffinöl | 14 |
| Parfüm, Antioxidantien, Konservierungsstoffe | q.s. |

| **Beispiel 6:** | |
|---|---|
| Paraffinöl | 40 |
| Natriumlaurethsulfat (25 %-ige Lösung in Wasser) | 38 |
| Cocamidopropylbetain (30 %-ige Lösung in Wasser) | 12 |
| Wasser | 9,4 |
| Parfüm, Antioxidantien, Konservierungsstoffe | q.s. |

| **Beispiel 7:** | |
|---|---|
| Natriumlaurethsulfat (25 %-ige Lösung in Wasser) | 35 |

(fortgesetzt)

| Beispiel 7: | |
|---|---|
| Sojaöl | 32 |
| Ricinusöl | 12 |
| Paraffinöl | 9 |
| Wasser | 6,6 |
| Decylglucosid | 5 |
| Parfüm, Antioxidantien, Konservierungsstoffe | q.s. |

| Beispiel 8: | |
|---|---|
| Paraffinöl | 60 |
| Natriumlaurethsulfat (25 %-ige Lösung in Wasser) | 39,4 |
| Titandioxid | 0,5 |
| Parfüm, Antioxidantien, Konservierungsstoffe | q.s. |

| Beispiel 9: | |
|---|---|
| Paraffinöl | 70 |
| Dinatriumlaurethsulfosuccinat (40 %-ige Lösung in Wasser) | 29,5 |
| Titandioxid | 0,5 |

## Patentansprüche

**1.** Verwendung von Mischungen, die bezogen auf das Gesamtgewicht der Zubereitungen

1 bis 50 Gew.-% eines oder mehrerer Tenside und
5 bis 60 Gew.-% Wasser und
25 bis 90 Gew.-% einer oder mehrerer Ölkomponenten sowie

gegebenenfalls weitere kosmetische oder pharmazeutische Hilfs-, Zusatz- und/oder Wirkstoffe enthalten, als kosmetische und/oder dermatologische Reinigungszubereitungen.

**2.** Verwendung von Zubereitungen nach Anspruch 1, wobei die Tenside aus der Gruppe der anionischen und amphoteren Tenside gewählt werden.

**3.** Verwendung von Zubereitungen nach Anspruch 1, wobei die verwendeten Tenside eine Kombination aus anionischen und/oder amphoteren Tensiden mit nicht-ionischen Tensiden darstellen.

**4.** Kosmetische und/oder dermatologische Reinigungszubereitungen, gekennzeichnet durch

einen Gehalt von 1 bis 50 Gew.-% eines oder mehrerer Tenside und
einen Gehalt von 5 bis 60 Gew.-% Wasser und

einen Gehalt von 25 bis 90 Gew.-% einer oder mehrerer Ölkomponenten sowie

einen zusätzlichen Gehalt, der den Gehalt der anderen Komponenten entsprechend vermindert, an mikronisierten, anorganischen Pigmenten, die

(a) eine mittlere Partikelgröße von weniger als 200 nm haben und deren Partikel
(b) sowohl hydrophile als auch lipophile Eigenschaften zeigen, die also amphiphilen Charakter besitzen und sich daher an der Grenzfläche Wasser/Öl anordnen und die
(c) gewählt werden aus der Gruppe der Metalloxide,

gegebenenfalls weitere kosmetische oder pharmazeutische Hilfs-, Zusatz- und/oder Wirkstoffe enthaltend.

**5.** Zubereitungen nach Anspruch 4, dadurch gekennzeichnet, daß die verwendeten anorganischen Pigmente oberflächlich beschichtet sind.